# EUROPEAN PATENT APPLICATION

(11) **EP 4 474 823 A1**
(43) Date of publication of application: **11.12.2024**
(21) Application number: 22922551.1
(22) Date of filing: 16.11.2022
(51) Int. Cl.: G01N 35/00

(54) **AUTOMATIC ANALYSIS DEVICE AND ALARM NOTIFICATION METHOD FOR AUTOMATIC ANALYSIS DEVICE**

(30) Priority: 31.01.2022 JP 2022012843
(71) Applicant: HITACHI HIGH-TECH CORPORATION, Tokyo 105-6409 (JP)
(72) Inventor: KUBOTA, Moe, Tokyo 105-6409 (JP); ITO, Kenta, Tokyo 105-6409 (JP)
(74) Representative: MERH-IP Matias Erny Reichl Hoffmann Patentanwälte PartG mbB
(86) International application number: PCT/JP2022/042615
(87) International publication number: WO 2023/145204

(57) **Abstract**

Provided is an automatic analyzer that notifies, on the basis of the status of examination work, a user of a non-urgent alarm using a time and a notification method that are suitable for the user. This automatic analyzer stores therein notification priority data 203d indicating the notification priority set for alarms and notification control time period data 203c indicating a notification control time period during which the alarm notification method is controlled for an alarm for which a predetermined notification priority is set. Using the notification priority data, the automatic analyzer determines whether or not an alarm corresponding to a detected abnormality is the alarm for which the predetermined notification priority is set. If the alarm is determined to be the alarm for which the predetermined notification priority is set, the automatic analyzer controls the notification method for the alarm depending on whether the time is within or outside the notification control time period for the alarm indicated by the notification control time period data.

## Description

### Technical Field

The present invention relates to an automatic analyzer and an alarm notification method for an automatic analyzer.

### Background Art

In an automatic analyzer that performs quantitative and qualitative analysis on a specific component of a sample such as blood or urine, when an abnormality occurs during operation, the occurrence of the abnormality is notified by a display on a device screen or an alarm sound and alarm lamp.

As an example of a technique related to alarm notification in the automatic analyzer, PTL 1 (WO2017/145601) discloses planning, based on information related to a device state of the automatic analyzer and a predetermined reference, at least two alarms including an abnormality occurrence alarm and a handling available alarm regarding each of abnormality items detected by the abnormality detection unit, the abnormality occurrence alarm being planned for notification to a user in a case where the abnormality occurs, and the handling available alarm being planned for notification to the user in a case where the automatic analyzer is in a device state where the automatic analyzer is available for handling the abnormality.

### Citation List

### Patent Literature

PTL 1: WO2017/145601

### Summary of Invention

### Technical Problem

In general, an alarm notified by an automatic analyzer is classified into levels of urgent stop, measurement impossible, attention, and the like according to a degree of affection of an occurred abnormality on a device or measurement. When an alarm due to abnormality occurrence of the urgent stop level or the measurement impossible level is notified, since the continuation of the sample measurement is not possible, it is necessary to take quick handling. On the other hand, when an alarm due to abnormality occurrence of the attention level is notified, since an operation of the device is not stopped, a user determines whether to continue the measurement.

In the alarm of the attention level, there is an alarm that does not need to stop the sample measurement for the purpose of handling. However, when the alarm is notified, the user needs to stop the work in order to check a content of the alarm. Interruption of the work of the user due to non-urgent alarm notification in a busy time and the like of examination work causes a decrease in work efficiency.

The automatic analyzer disclosed in PTL 1 can preset, for each alarm, a determination criterion for determining that the automatic analyzer is in a device state where the automatic analyzer is available for handling the abnormality, and the user can handle the alarm in a timely manner by performing alarm notification in two stages, that is, an abnormality occurrence time and a handling available time.

On the other hand, there is a problem that a status of the user at the time of alarm notification and an urgency for each alarm are not considered. For example, in a busy time of examination work, the user needs to quickly handle the alarm that affects the continuation of measurement. Since it is sufficient to handle a non-urgent alarm in a time suitable for the user, if the notification is given in a non-busy time, the number of alarms in the busy time can be reduced, and the user can focus on the examination work.

In addition, when an alarm is generated while a user who is not skilled in using the device is using the device, the user cannot handle the alarm, and thus when an inquiry is made to another user, the examination work may be interrupted. Therefore, in the case of the non-urgent alarm, it is possible to reduce the time and effort of an unnecessary inquiry and the like by notifying the user within a working time of the user who can handle the alarm.

An object of the invention is to provide an automatic analyzer that notifies, based on a status of examination work, a user of a non-urgent alarm using a time and a notification method that are suitable for the user.

### Solution to Problem

In order to achieve the above object, an automatic analyzer according to one embodiment of the invention, which performs quantitative and qualitative analysis on a specific component of a sample, includes: an abnormality detection unit configured to detect an abnormality; an alarm control unit configured to control notification of an alarm at the abnormality detected by the abnormality detection unit; an alarm notification unit configured to notify a user of an alarm; and a storage unit configured to store data for the alarm control unit to control the notification of the alarm. The storage unit stores notification priority data indicating a notification priority set for an alarm, and notification control time period data indicating a notification control time period during which a notification method is controlled for the alarm for which a predetermined notification priority is set. When it is determined that an alarm corresponding to the abnormality detected by the abnormality detection unit is the alarm for which the predetermined notification priority is set based on the notification priority data, the alarm control unit controls a notification method of the alarm by the alarm notification unit depending on whether a time is within or outside a notification control time period for the alarm indicated by the notification control time period data.

### Advantageous Effects of Invention

According to the invention, based on a status of examination work, a user is notified of a non-urgent alarm using a time and a notification method that are suitable for the user, and thus work efficiency of an entire examination room can be improved. Other objects and novel features will become apparent from description of the present specification and the accompanying drawings.

### Brief Description of Drawings

[FIG. 1] FIG. 1 is a schematic configuration diagram of an automatic analyzer.
[FIG. 2] FIG. 2 is a functional block diagram of an operation unit PC.
[FIG. 3] FIG. 3 is a diagram showing an example of an alarm list screen.
[FIG. 4] FIG. 4 is a diagram showing an example of an alarm notification priority setting screen.
[FIG. 5] FIG. 5 is a diagram showing an example of an alarm notification control setting screen.
[FIG. 6] FIG. 6 is a diagram showing an example of an alarm notification control time period setting screen.
[FIG. 7] FIG. 7 is a flowchart from abnormality detection to alarm notification when alarm notification control is enabled.
[FIG. 8] FIG. 8 is a diagram showing an example of a user information setting screen.
[FIG. 9] FIG. 9 is a flowchart from abnormality detection to alarm notification when an alarm notification restriction is enabled.

### Description of Embodiments

Embodiments of the invention will be described with reference to the drawings. In the drawings used in the specification, the same or corresponding components are denoted by the same or similar reference numerals, and repeated descriptions of the components may be omitted.

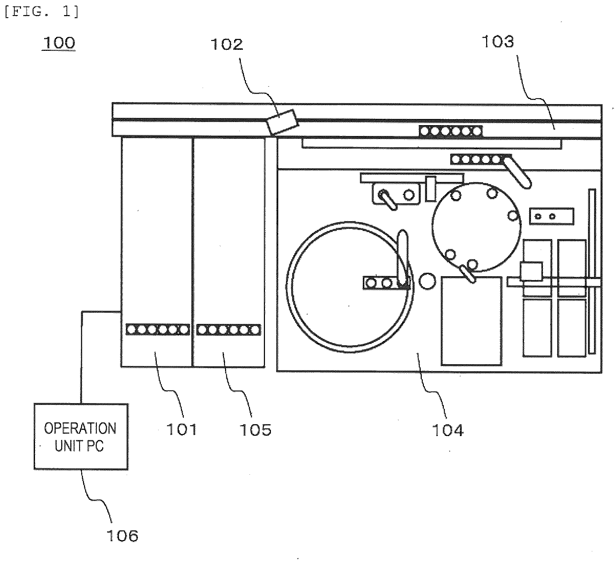

First, an automatic analyzer according to an embodiment of the invention will be described with reference to FIG. 1. FIG. 1 is a schematic configuration diagram of the automatic analyzer according to the embodiment. An automatic analyzer 100 includes a sample carry-in unit 101, a sample information reading unit 102, a transport line 103, an analysis unit 104, a sample housing unit 105, an operation unit PC 106, and the like.

The sample carry-in unit 101 is a unit for carrying a sample container housing a patient sample, a control sample, and an accuracy control sample in a sample rack in the automatic analyzer 100.

A tag such as a bar code or an RFID indicating identification information is attached to a sample container, and the sample information reading unit 102 reads the identification information to identify a sample. The read information of the sample is sent to the operation unit PC 106.

The transport line 103 is a unit that transports the sample rack supplied from the sample carry-in unit 101 to the target analysis unit 104 in accordance with an analysis request from a user, and transports the sample after the analysis is completed to the sample housing unit 105.

The analysis unit 104 is implemented by an analysis device that performs biochemical analysis, immune analysis, electrolyte analysis, and the like, and is a unit that performs analysis on the sample. In the embodiment, an automatic analyzer including one analysis device is described as an example, and an automatic analyzer including one or more analysis devices may be used. In addition, a configuration of the analysis unit 104 is not particularly limited, and in a case of a configuration including a plurality of analysis devices, a combination of the same analysis devices or different analysis devices may be used.

The sample housing unit 105 is a unit that houses the sample after analysis is completed.

The operation unit PC 106 is a computer including a CPU and a memory. The operation unit PC 106 is communicably connected to each device of the automatic analyzer 100 via a wired or wireless network line, controls an operation of each device of the automatic analyzer 100 based on a program or data input by the user, and monitors a device state and an analysis status of the sample. The operation unit PC 106 may be connected to a terminal such as a PC, a tablet, or a mobile held by a high-order host system that controls the entire hospital or examination room system or a user who operates the automatic analyzer 100.

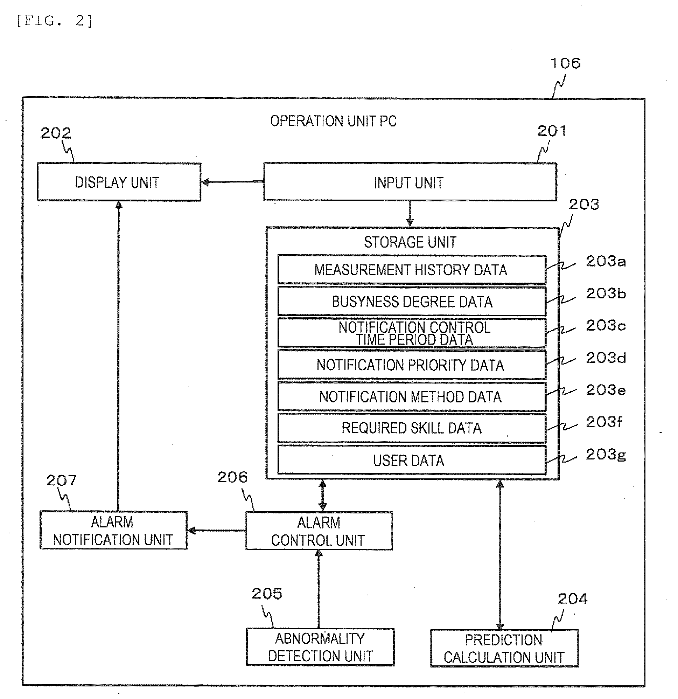

FIG. 2 is a functional block diagram of the operation unit PC 106 provided in the automatic analyzer 100. In FIG. 2, portions related to alarm functions in the operation unit PC 106 are extracted and shown, and other control functions are omitted. The operation unit PC 106 includes an input unit 201, a display unit 202, a storage unit 203, a prediction calculation unit 204, an abnormality detection unit 205, an alarm control unit 206, and an alarm notification unit 207.

The input unit 201 is a device such as a keyboard, a mouse, or a touch panel through which the user inputs an instruction or information to the automatic analyzer 100.

The display unit 202 is a display that displays various operation screens, alarm information, and the like. When the display unit 202 is a touch panel type, the display unit 202 also functions as the input unit 201.

The storage unit 203 stores a program for controlling the automatic analyzer 100, measurement history data 203a, busyness degree data 203b, notification control time period data 203c, notification priority data 203d, notification method data 203e, required skill data 203f, and user data 203g. The measurement history data 203a is data such as a reading date and a reading time of the sample read by the sample information reading unit 102 of the automatic analyzer 100. The busyness degree data 203b is data of a busyness degree and a busy time period of each time predicted by the prediction calculation unit 204 described later. The notification control time period data 203c is data of a notification control time period set based on the busy time period predicted by the prediction calculation unit 204 and a notification control time period set by the user. The notification priority data 203d is data of a notification priority set for each alarm. The notification method data 203e is data of a notification method of an alarm set by the user. The required skill data 203f is data of a skill required for handling the alarm set for the alarm by the user. The user data 203g is data such as a user ID, a user name, a day of working, a working time, and a holding skill of the user who uses the automatic analyzer 100.

The prediction calculation unit 204 predicts a busyness degree of each time period based on the data of the measurement history data 203a stored in the storage unit 203, and sets a busy time period of examination work based on the predicted busyness degree. A method of predicting the busyness degree will be described later.

The abnormality detection unit 205 detects an abnormality in the automatic analyzer 100 based on information from a sensor and the like attached to the automatic analyzer 100.

The alarm control unit 206 determines a notification time and a notification method of an alarm based on the information stored in the storage unit 203 for each abnormality item detected by the abnormality detection unit 205.

The alarm notification unit 207 notifies the user of the alarm by displaying an alarm screen for signaling occurrence of an abnormality on the display unit 202, emitting an alarm sound from a speaker provided in the automatic analyzer 100, or turning on an alarm lamp.

The method of predicting the busyness degree by the prediction calculation unit 204 will be described. Based on the data of the measurement history data 203a stored in the storage unit 203, the number of measured samples in the past is totaled for each day of the week and each time period, and a predicted value of the number of samples in each time period for a day of the week is calculated based on an average value. A time period in which the number of samples is predicted to be the largest in one day is set to a busyness degree of 100%. The busyness degree (%) is calculated by comparing the number of samples in the time period in which the number of samples is predicted to be the largest with the predicted number of samples in each time period. The busyness degree is calculated by the following equation.

Busyness degree (%) = the predicted number of samples in certain time period/the number of samples in time period in which the number of samples is predicted to be largest in one day × 100

For example, when the predicted number of samples in the time period in which the number of samples is predicted to be the largest is 300 and the predicted number of samples from 12:00 to 13:00 is 150, the busyness degree from 12:00 to 13:00 is calculated as 50%.

The information used for the prediction is not limited to the number of measured samples in the past, and an element that may affect a busy status may be reflected in a result calculated above.

As an example, a case will be described in which the number of workers performing the examination work is used as an element that may affect the busy status. In general, shift control is often adopted in work in an examination room, and the number of workers may vary depending on a time period. Therefore, by reflecting the number of workers in each time in the busyness degree predicted based on the number of samples, it is possible to perform prediction more suitable for an actual status of the examination room. The number of workers in each time can be calculated based on the working time of the user stored in the user data 203g of the storage unit 203.

For example, it is assumed that there are two workers at 7:00 to 8:00 and there are four workers at 8:00 to 9:00. When it is assumed that the work for 100 samples is performed in each of the time periods, burden per person in 7: 00 to 8: 00 is 50 samples, whereas the burden per person in 8: 00 to 9: 00 is 25 samples, and the burden per worker in the former is twice the burden in the latter. Therefore, a coefficient is set according to the number of workers. For example, when the coefficient in the case of work by four persons is 1, the coefficient in the case of work by two persons is 2. By multiplying the number of samples per hour by the coefficient, the number of samples per hour reflecting the number of workers is calculated as follows.
7:00 to 8: 00 (in the case of work by two persons): 100 × 2 = 200 samples
8:00 to 9: 00 (in the case of work by four persons): 100 × 1 = 100 samples

From the above, the busyness degree in 8:00 to 9:00 is calculated as 50% of the busyness degree with respect to the busyness degree in 7:00 to 8:00. Similarly, by multiplying the predicted number of samples in each time by a coefficient corresponding to the number of workers, it is possible to reflect, in the busyness degree, the affection of the change in the work amount depending on the number of workers.

The information that may affect the busy status is not limited to the above example, and includes various examples.

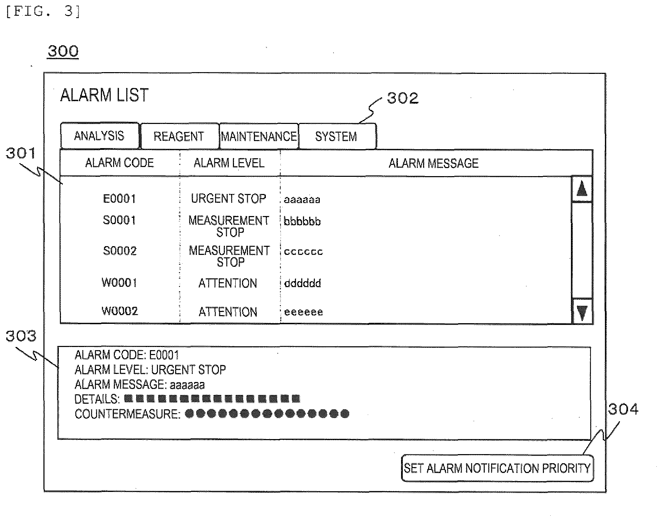

FIG. 3 is a diagram showing an example of an alarm list screen. An alarm list screen 300 shown in FIG. 3 is a screen for displaying all alarms notified by the automatic analyzer 100. The alarm list screen 300 includes an alarm list part 301, an alarm category selection part 302, an alarm content display part 303, and an alarm notification priority setting screen display button 304.

The alarm list part 301 displays an alarm code, an alarm level, and an alarm message for all the alarms notified by the automatic analyzer 100. The alarm code is a code for uniquely specifying an alarm. The alarm level is set according to the importance of the alarm. The alarm message is a message displayed on the display unit 202 to make the user know a situation. In addition, the alarm is classified into categories for each related function, and the user checks the alarm of each category by switching a tab of the alarm category selection part 302. In this example, the alarm category selection part 302 is classified into four categories of "analysis", "reagent", "maintenance", and "system". Accordingly, the user can efficiently search for a target alarm from among a large number of alarms. When the user selects an alarm whose detailed content is desired to be checked, an alarm code, an alarm level, an alarm message, details, and a countermeasure of the alarm are displayed in the alarm content display part 303.

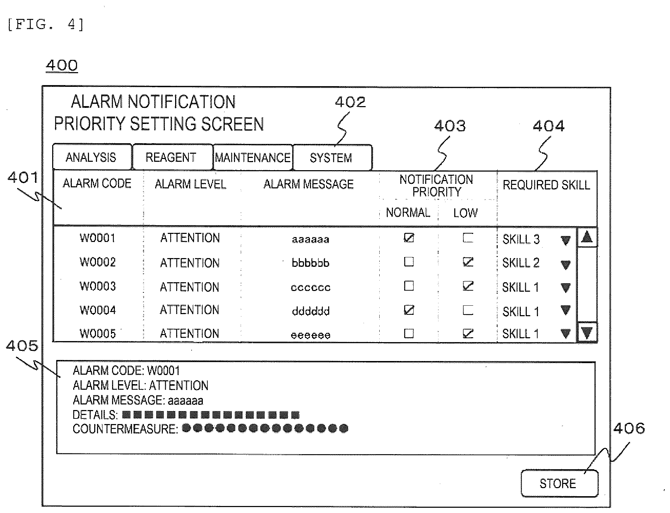

When the user presses the alarm notification priority setting screen display button 304, an alarm notification priority setting screen is displayed. FIG. 4 shows an example of the alarm notification priority setting screen. An alarm notification priority setting screen 400 includes an alarm list part 401, an alarm category selection part 402, a notification priority setting part 403, a required skill setting part 404, an alarm content display part 405, and a store button 406.

The alarm list part 401 displays an alarm, for which a notification priority can be set by the user, among the alarms output from the automatic analyzer 100. Here, the alarm for which the notification priority can be set by the user is an alarm of an attention level at which the user determines whether the analysis is continued without stopping the sample measurement even when an abnormality occurs. The alarm list part 401 displays an alarm code, an alarm level, and an alarm message for the alarm whose alarm level is the attention level. The user can check the alarm of each category by switching a tab of the alarm category selection part 402. When the user selects an alarm whose detailed content is desired to be checked, an alarm code, an alarm level, an alarm message, details, and a countermeasure of the alarm are displayed in the alarm content display part 405.

The notification priority of the alarm can be set for each alarm in the notification priority setting part 403. In the embodiment, an example is shown in which one of two priorities of "normal" and "low" is set as the notification priority of the alarm based on the urgency. When an alarm is generated in the automatic analyzer 100, the alarm for which the notification priority is set to "normal" is immediately notified to the user. For an alarm for which the notification priority is set to "low", a notification time and a notification method for notifying the user of the alarm are determined by the alarm control unit 206 based on a notification control time period, a notification method, and the like set in an alarm notification control setting screen described later. A default setting value of the notification priority may be set to "normal", and the setting value of the notification priority for each alarm may be changed, as necessary. In addition, in the example shown in FIG. 4, an example is shown in which the user sets the notification priority for each alarm one by one, but in order to reduce the time and effort of the setting by the user, a button and the like, which can collectively set the notification priority of all the alarms in a specific category, may be provided.

The user sets, to "low", a notification priority of an alarm for which notification is not desired to be received in a specific time period or an alarm for which a notification method is desired to be restricted. For example, since the measurement of the sample is not affected, the notification priority of the alarm is set to "low" for the alarm considered by the user that the urgency is low. Examples of the alarm that does not affect the measurement include an alarm notified when a capacity of data stored in the operation unit PC 106 reaches a reference value, and an alarm recommended for maintenance. Since any alarm does not immediately stop the device or interrupt the measurement, it is an alarm without any problem if the alarm is handled at a time suitable for the work of the user.

In the required skill setting part 404, the user sets a skill level required for handling each alarm. In the embodiment, an example is shown in which the skill level is set in three levels (the larger the numerical value is, the higher the level is). However, a form of dividing the skill level is not particularly limited.

The store button 406 is a button for storing setting contents input by the user on the alarm notification priority setting screen 400. By pressing the store button 406, processing of storing information set by the notification priority setting part 403 as the notification priority data 203d of the storage unit 203 and storing information set by the required skill setting part 404 as the required skill data 203f of the storage unit 203 is executed.

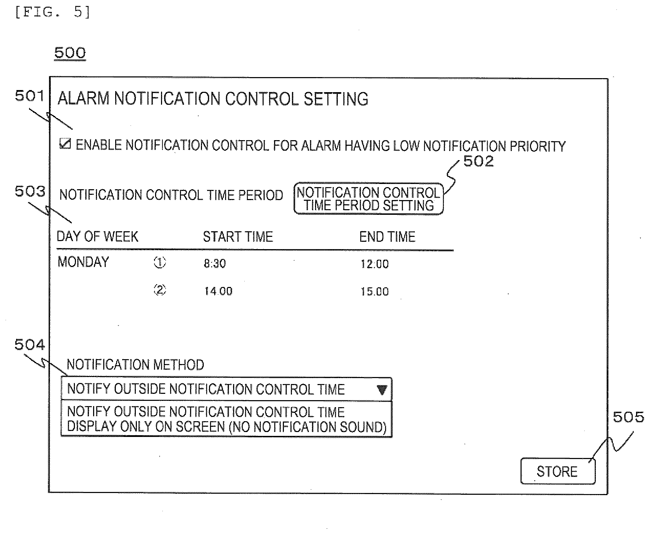

FIG. 5 is a diagram showing an example of the alarm notification control setting screen. An alarm notification control setting screen 500 is a screen for setting a notification control time period and a notification method for an alarm set to have a low notification priority. The alarm notification control setting screen 500 includes an alarm notification control switching part 501, an alarm notification control time period setting screen display button 502, a notification control time period list part 503, a notification method setting part 504, and a store button 505.

The alarm notification control switching part 501 is a button for the user to switch between enabling and disabling alarm notification control. Here, when the user checks a check box, the alarm notification control is enabled, and when the user unchecks the check box, the alarm notification control is disabled. The alarm notification control is control, for an alarm set to have a low notification priority, for notifying the user of the alarm according to a set notification control time period and notification method. When the alarm notification control is disabled, the alarm is immediately notified to the user regardless of the setting of the notification priority.

When the user presses the alarm notification control time period setting screen display button 502, an alarm notification control time period setting screen for the user to set an alarm notification control time period is displayed. The alarm notification control time period setting screen will be described later. A day of the week, a start time, and an end time of all the notification control time periods set in the alarm notification control time period setting screen are displayed in the notification control time period list part 503. The setting number of the notification control time periods may be one or more.

The notification method setting part 504 selectively sets, from a plurality of predetermined candidates, a notification method in a case where an alarm set to have a low notification priority is generated in the notification control time period. In the embodiment, a case is exemplified in which either "notify outside notification control time" or "display only on screen (no notification sound)" is selected as the alarm set to have a low notification priority. For example, when the notification method is set to "notify outside notification control time", if an alarm set to have a low notification priority is generated in the notification control time period, the automatic analyzer 100 suspends the notification of the alarm in the notification control time period and notifies the alarm after the notification control time period. When a plurality of alarms are generated in the notification control time period, the alarms are collectively notified. When the notification method is set to "display only on screen (no notification sound)", if an alarm set to have a low notification priority is generated in the notification control time period, the automatic analyzer 100 displays a screen for signaling the generation of the alarm only on the display unit 202 without generating a notification sound. whereas the alarm notifies the alarm by a notification sound and a screen outside the notification control time period. The notification method is not limited to the above example, and includes various examples.

The store button 505 is a button for storing the notification control time period and the notification method set in the alarm notification control setting screen 500. By pressing the store button 505, processing of storing notification control time period information set in the alarm notification control time period setting screen as the notification control time period data 203c of the storage unit 203 and information set by the notification method setting part 504 as the notification method data 203e of the storage unit 203 is executed.

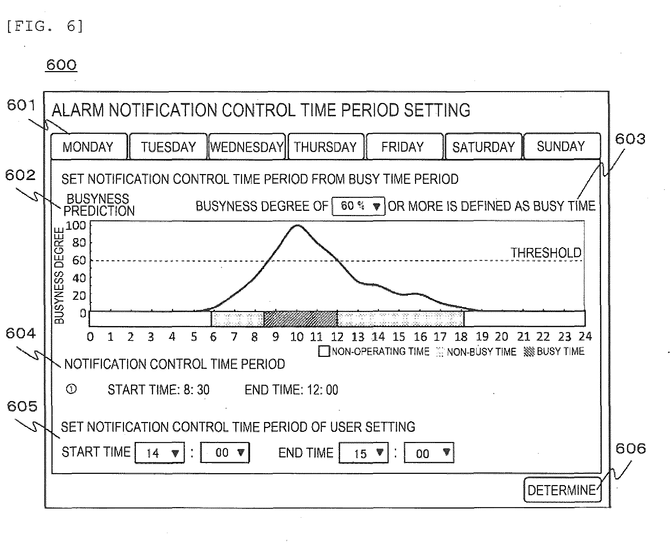

FIG. 6 is a diagram showing an example of the alarm notification control time period setting screen. An alarm notification control time period setting screen 600 includes a day of week selection part 601, a busyness prediction display part 602, a busyness degree threshold setting part 603, a notification control time period display part 604, a user setting part 605, and a determine button 606.

The day of week selection part 601 is used to switch a screen for setting a notification control time period for each day of the week. In the embodiment, an example is shown in which a notification control time period of Monday is set.

The busyness prediction display part 602 displays a busyness degree predicted by the prediction calculation unit 204 such that the busyness degree for each time period can be easily recognized. Accordingly, when the user sets a threshold of the busyness degree in the busyness degree threshold setting part 603 to be described later, the user can perform the setting while checking the busy status for each time period. Here, the change in the busyness degree for each time period is displayed in a line graph, but the display form is not limited to the form shown in FIG. 6.

The user sets the threshold of the busyness degree in the busyness degree threshold setting part 603. The prediction calculation unit 204 determines, as a busy time period, a time period in which a predicted value of the busyness degree exceeds the set threshold. Here, an example in which the threshold of the busyness degree is set to 60% is shown, and 8: 30 to 12: 00 is the busy time period. Although FIG. 6 shows a case where the busy time period is only one time period, there may be a plurality of time periods. When the threshold of the busyness degree is not set, the busy time period is not calculated.

The time period determined to be the busy time period is displayed in the notification control time period display part 604 as a notification control time period. By setting the busy time period predicted from a past measurement history as the notification control time period, the notification control time period is automatically reset even in a case where the tendency of increase or decrease and the like in the number of samples is changed, and thus it is possible to save the time and effort for the user to change the setting. In addition, by predicting a busy status for each day of the week, it is possible to cope with a case where there is a difference in examination work or the number of samples depending on the day of the week.

The user setting part 605 is used when the user sets the notification control time period. The user inputs a start time and an end time of the notification control time period to the user setting part 605.

The determine button 606 is a button for registering, in the notification control time- period list part 503 of the alarm notification control setting screen 500, the notification control time period set in the alarm notification control time period setting screen 600. In addition, when the user presses the determine button 606, the alarm notification control time period setting screen 600 is closed, and the screen returns to the alarm notification control setting screen 500.

A setting procedure of the notification control time period using the alarm notification control setting screen 500 and the alarm notification control time period setting screen 600 is as follows.

First, the user presses the alarm notification control time period setting screen display button 502 of the alarm notification control setting screen 500 to display the alarm notification control time period setting screen 600.

### (1) A case where the busy time period is set as the notification control time period

The user sets the threshold of the busyness degree by the busyness degree threshold setting part 603. When the threshold of the busyness degree is set, the time period determined as the busy time period by the prediction calculation unit 204 is displayed as the notification control time period in the notification control time period display part 604. Thereafter, when the determine button 606 is pressed, the alarm notification control time period setting screen 600 is closed, and the notification control time period is registered in the notification control time period list part 503 of the alarm notification control setting screen 500.

### (2) A case where a notification control time period of user setting is set

The user inputs the start time and the end time of the notification control time period to the user setting part 605. Thereafter, when the determine button 606 is pressed, the notification control time period input to the user setting part 605 is registered in the notification control time period list part 503 of the alarm notification control setting screen 500.

A busy time period can be set as the notification control time period, or a notification control time period of user setting can be set as the notification control time period. As the notification control time period, only one or both of the notification control time periods may be set.

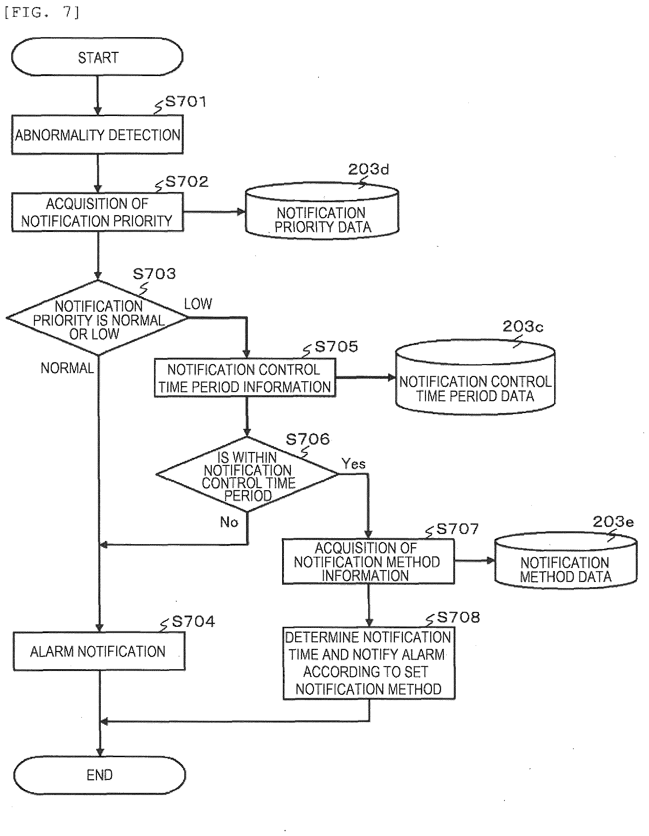

A flow from abnormality detection to alarm notification when the alarm notification control is enabled in the automatic analyzer 100 will be described with reference to FIG. 7.

When the abnormality detection unit 205 detects an abnormality occurring in the automatic analyzer 100 (S701), the alarm control unit 206 acquires the notification priority data 203d stored in the storage unit 203 for an alarm corresponding to the abnormality, and proceeds to a determination step of a notification priority (S703).

### (1) A case of an alarm whose notification priority is normal

As a result of the determination of the notification priority (S703), when the notification priority is normal, the alarm control unit 206 sends alarm information to the alarm notification unit 207 and notifies the user of the alarm (S704).

### (2) A case of an alarm whose notification priority is low

As a result of the determination of the notification priority (S703), when the notification priority is low, the processing proceeds to a step of acquiring notification control time period information (S705), and the alarm control unit 206 acquires the notification control time period data 203c stored in the storage unit 203. Subsequently, the processing proceeds to a step of determining the notification control time period (S706). When a time at which the alarm is generated is outside the notification control time period in step S706, the alarm control unit 206 sends the alarm information to the alarm notification unit 207 and notifies the user of the alarm (S704). On the other hand, when the time at which the alarm is generated is within the notification control time period in step S706, the alarm control unit 206 acquires the notification method data 203e stored in the storage unit 203 (S707). Thereafter, the alarm control unit 206 determines a notification time of the alarm and notifies the alarm according to the set notification method (S708).

Accordingly, by setting the notification control time period and the notification method for the alarm having a low notification priority, the alarm can be notified to the user using a time and a notification method that are suitable for the user. Since the user can focus on the examination work without being bothersome by the alarm having a low notification priority, the work efficiency of the examination work can be improved.

Subsequently, an example in which notification control of the alarm is performed according to a holding skill of the user will be described.

For example, when an alarm is generated while a user who is not skilled in using the device is using the device, the user cannot handle the alarm, and thus it may be necessary to make an inquiry and the like to another user. Therefore, in a case of a non-urgent alarm, it is expected to reduce the time and effort of an unnecessary inquiry and the like by setting a restriction according to a holding skill of a working user.

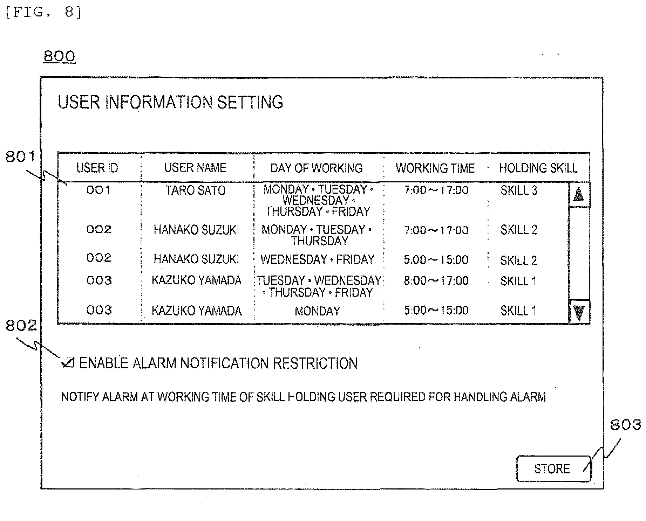

FIG. 8 is a diagram showing an example of a user information setting screen. A user information setting screen 800 includes a user information registration part 801, an alarm notification restriction switching part 802, and a store button 803.

The user registers, in the user information registration part 801, a user ID, a user name, a day of working, a working time, and a holding skill of the user who uses the automatic analyzer 100.

The alarm notification restriction switching part 802 is used to switch between enabling and disabling an alarm notification restriction according to the holding skill of the working user. For example, when the user checks a check box, the notification restriction is enabled, and when the user unchecks the check box, the notification restriction is disabled. In a case where the notification restriction is enabled, when an alarm set to have a low notification priority is generated, the alarm control unit 206 compares a required skill set for the alarm with the registered holding skill of the user, thereby determining whether the user who can handle the alarm is working, and determines whether to suspend the notification of the alarm. For example, when a skill holder required for handling the alarm is outside the working time, the alarm control unit 206 suspends the notification of the alarm until the next working time of the skill holder required for handling the alarm is reached.

For the notification restriction, user information shown in FIG. 8 will be described as an example. For example, when an alarm of a required skill 3 is generated at 5:00 on Monday, since the holding skill of the user working at the time is "1", the user working at the time cannot handle the alarm. Since the next working time of the user having the skill 3 is 7:00 to 17:00 on Monday, the alarm is notified after 7:00 on Monday.

The store button 803 is a button for executing processing of storing, in the user data 203g of the storage unit 203, the information set in the user information setting screen 800.

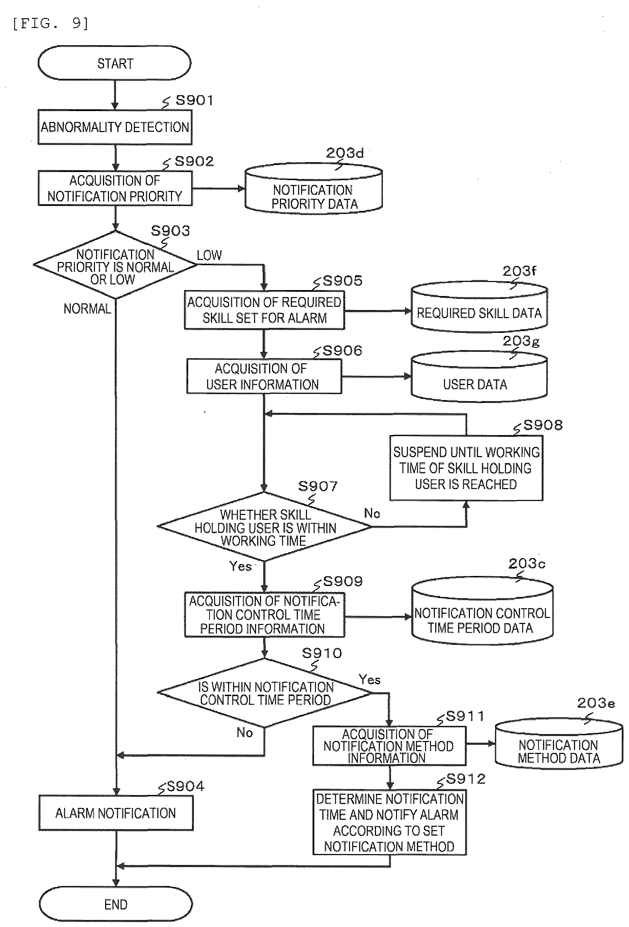

A flow from abnormality detection to alarm notification, when a notification restriction based on a user skill is enabled, will be described with reference to FIG. 9.

When the abnormality detection unit 205 detects an abnormality occurring in the automatic analyzer 100 (S901), the alarm control unit 206 acquires the notification priority data 203d stored in the storage unit 203 for an alarm corresponding to the abnormality, and proceeds to a determination step of a notification priority (S903).

### (1) A case of an alarm whose notification priority is normal

As a result of the determination of the notification priority (S903), when the notification priority is normal, the alarm control unit 206 sends alarm information to the alarm notification unit 207 and notifies the user of the alarm (S904).

### (2) A case of an alarm whose notification priority is low

As a result of the determination of the notification priority (S903), when the notification priority is low, the processing proceeds to a step of acquiring a required skill set for the alarm (S905), and the alarm control unit 206 acquires the required skill of the alarm from the required skill data 203f stored in the storage unit 203. Subsequently, the processing proceeds to a step of acquiring the user information (S906), and the alarm control unit 206 acquires the day of working, the working time, and the holding skill of the user from the user data 203g stored in the storage unit 203. Thereafter, the processing proceeds to a step of determining whether a user (hereinafter, referred to as a skill holding user) holding a skill required for handling the alarm is within the working time (S907). When the skill holding user is within the working time, the processing proceeds to the step of acquiring the notification control time period information, and the alarm control unit 206 acquires a notification control time period from the notification control time period data 203c stored in the storage unit 203 (S909). When the skill holding user is outside the working time, the alarm control unit 206 suspends the notification of the alarm until the next working time of the skill holding user is reached (S908), and then proceeds to the step of acquiring the notification control time period information (S909). Subsequently, in a step of determining the notification control time period (S910), when a time at which the alarm is to be notified is outside the notification control time period, the alarm control unit 206 sends the alarm information to the alarm notification unit 207 and notifies the user of the alarm (S904). When the time at which the alarm is to be notified is within the notification control time period, the alarm control unit 206 acquires the notification method data 203e stored in the storage unit 203 (S911). Thereafter, the alarm control unit 206 determines a notification time of the alarm and notifies the alarm according to the set notification method (S912).

As described above, by restricting the notification of the alarm according to the holding skill of the user, it is possible to notify the alarm at a time at which the user can cope with the alarm, so that the burden on the user is reduced, and the work efficiency of the examination work can be improved.

Effects of the embodiment configured as described above will be described.

When the alarm is notified, the user needs to stop the work in order to check a content of the alarm. In a busy time period of examination work, a situation in which a user who is not skilled in using the device uses the device, and the like, notification of a non-urgent alarm is a burden on the user and causes a decrease in work efficiency.

On the other hand, in the embodiment, the user sets the notification priority, the notification control time period, the notification method, and the like of the alarm according to operations of facilities. Then, when an alarm is generated in the notification control time period, the alarm control unit determines a notification time and a notification method based on the notification priority of the alarm. Accordingly, since the alarm can be notified according to the operations of the facilities, the alarm can be notified to the user using a time and a notification method that are suitable for the user. In addition, since the user does not need to interrupt the examination work for the non-urgent alarm handling, the work efficiency of the entire examination room can be improved.

The invention is not limited to the embodiments described above, and includes various modifications. For example, the embodiments described above have been described in detail to facilitate understanding of the invention, and the invention is not necessarily limited to those including all the configurations described above. For example, when only one notification control method in the notification control time period is applied (for example, when only the notification of the alarm is suspended), it is not necessary to store the notification method data 203e in the storage unit 203. In addition, in the embodiment, an example in which information for controlling the notification of the alarm is set and registered in the operation unit PC 106 has been described, but the invention is not limited thereto. When a plurality of automatic analyzers are disposed in a clinical examination room and operations thereof are managed, data thereof may be stored from a management server connected to an operation unit PC of each automatic analyzer. In this case, it is also possible to change contents of data to be stored according to an operation of the clinical examination room. For example, a work date and time and a holding skill of the user working in the clinical examination room may be registered as the user data 203g without specifying the automatic analyzer.

A part of a configuration according to one embodiment can be replaced with a configuration according to another embodiment, and a configuration according to another embodiment can also be added to a configuration according to one embodiment. A part of a configuration according to each embodiment may be added to, deleted from, or replaced with another configuration.

### Reference Signs List

100: automatic analyzer
101: sample carry-in unit
102: sample information reading unit
103: transport line
104: analysis unit
105: sample housing unit
106: operation unit PC
201: input unit
202: display unit
203: storage unit
203a: measurement history data
203b: busyness degree data
203c: notification control time period data
203d: notification priority data
203e: notification method data
203f: required skill data
203g: user data
204: prediction calculation unit
205: abnormality detection unit
206: alarm control unit
207: alarm notification unit
300: alarm list screen
301: alarm list part
302: alarm category selection part
303: alarm content display part
304: alarm notification priority setting screen display button
400: alarm notification priority setting screen
401: alarm list part
402: alarm category selection part
403: notification priority setting part
404: required skill setting part
405: alarm content display part
406: store button
500: alarm notification control setting screen
501: alarm notification control switching part
502: alarm notification control time period setting screen display button
503: notification control time period list part
504: notification method setting part
505: store button
600: alarm notification control time period setting screen
601: day of week selection part
602: busyness prediction display part
603: busyness degree threshold setting part
604: notification control time period display part
605: user setting part
606: determine button
800: user information setting screen
801: user information registration part
802: alarm notification restriction switching part
803: store button

## Claims

1. An automatic analyzer that performs quantitative and qualitative analysis on a specific component of a sample, the automatic analyzer comprising:
an abnormality detection unit configured to detect an abnormality;
an alarm control unit configured to control notification of an alarm at the abnormality detected by the abnormality detection unit;
an alarm notification unit configured to notify a user of an alarm; and
a storage unit configured to store data for the alarm control unit to control the notification of the alarm, wherein
the storage unit stores notification priority data indicating a notification priority set for an alarm, and notification control time period data indicating a notification control time period during which a notification method is controlled for the alarm for which a predetermined notification priority is set, and
when it is determined that an alarm corresponding to the abnormality detected by the abnormality detection unit is the alarm for which the predetermined notification priority is set based on the notification priority data, the alarm control unit controls a notification method of the alarm by the alarm notification unit depending on whether a time is within or outside a notification control time period for the alarm indicated by the notification control time period data.

2. The automatic analyzer according to claim 1, wherein
when it is determined that the alarm corresponding to the abnormality detected by the abnormality detection unit is the alarm for which the predetermined notification priority is set based on the notification priority data, the alarm control unit suspends notification of the alarm to a user within the notification control time period for the alarm and causes the alarm notification unit to notify a user of the alarm outside the notification control time period for the alarm.

3. The automatic analyzer according to claim 1, wherein
the storage unit stores, for the alarm for which the predetermined notification priority is set, notification method data indicating the notification method of the alarm in the notification control time period, and
when it is determined that the alarm corresponding to the abnormality detected by the abnormality detection unit is the alarm for which the predetermined notification priority is set based on the notification priority data, the alarm control unit causes the alarm notification unit to notify a user of the alarm by a notification method, which is indicated by the notification method data within the notification control time period for the alarm, different from a notification method outside the notification control time period for the alarm.

4. The automatic analyzer according to claim 1, wherein
a busy time period predicted based on a past measurement history is set as the notification control time period in the notification control time period data.

5. The automatic analyzer according to claim 4, further comprising:
a prediction calculation unit configured to predict a busyness degree, wherein
the storage unit stores measurement history data indicating the past measurement history,
the prediction calculation unit predicts the busyness degree based on the number of samples in each time period for each day of a week predicted based on the measurement history data, and
a time period in which the busyness degree is equal to or larger than a predetermined threshold is set as the busy time period.

6. The automatic analyzer according to claim 1, wherein
in a case where notification control of an alarm is set to be disabled, even though it is determined that the alarm corresponding to the abnormality detected by the abnormality detection unit is the alarm for which the predetermined notification priority is set based on the notification priority data, the alarm control unit does not perform the notification control of the alarm depending on whether a time is within or outside the notification control time period for the alarm indicated by the notification control time period data.

7. The automatic analyzer according to claim 1, wherein
the storage unit stores required skill data indicating a required skill level set as a skill level required for handling an alarm, and user data indicating user information including at least a day of working, a working time, and a holding skill of a user, and
when it is determined that the alarm corresponding to the abnormality detected by the abnormality detection unit is the alarm for which the predetermined notification priority is set based on the notification priority data, the alarm control unit suspends notification of the alarm to a user until a working time of a user having a holding skill satisfying the required skill level set for the alarm is reached.

8. The automatic analyzer according to claim 1, wherein
the predetermined notification priority is a notification priority for an alarm that does not require to stop sample measurement and is a notification priority evaluated as having low urgency.

9. An alarm notification method for an automatic analyzer that performs quantitative and qualitative analysis on a specific component of a sample, the alarm notification method comprising:
storing in advance notification priority data indicating a notification priority set for an alarm and notification control time period data indicating a notification control time period during which a notification method is controlled for the alarm for which a predetermined notification priority is set;
determining whether an alarm corresponding to a detected abnormality is the alarm for which the predetermined notification priority is set by using the notification priority data; and
controlling, when it is determined that the alarm corresponding to the detected abnormality is the alarm for which the predetermined notification priority is set, the notification method of the alarm depending on whether a time is within or outside the notification control time period for the alarm indicated by the notification control time period data.

10. The alarm notification method according to claim 9, further comprising:
when it is determined that the alarm corresponding to the detected abnormality is the alarm for which the predetermined notification priority is set, suspending notification of the alarm to a user within the notification control time period for the alarm and notifying a user of the alarm outside the notification control time period for the alarm.

11. The alarm notification method according to claim 9, further comprising:
storing in advance, for the alarm for which the predetermined notification priority is set, notification method data indicating the notification method of the alarm in the notification control time period; and
when it is determined that the alarm corresponding to the detected abnormality is the alarm for which the predetermined notification priority is set, notifying a user of the alarm by a notification method, which is indicated by the notification method data within the notification control time period for the alarm, different from a notification method outside the notification control time period for the alarm.

12. The alarm notification method according to claim 9, wherein
a busy time period predicted based on a past measurement history is set as the notification control time period in the notification control time period data.

13. The alarm notification method according to claim 9, further comprising:
storing in advance required skill data indicating a required skill level set as a skill level required for handling an alarm, and user data indicating user information including at least a day of working, a working time, and a holding skill of a user; and
when it is determined that the alarm corresponding to the detected abnormality is the alarm for which the predetermined notification priority is set, suspending notification of the alarm to a user until a working time of a user having a holding skill satisfying the required skill level set for the alarm is reached.

14. The alarm notification method according to claim 9, wherein
the predetermined notification priority is a notification priority for an alarm that does not require to stop sample measurement and is a notification priority evaluated as having low urgency.
